# EUROPEAN PATENT SPECIFICATION

(11) **EP 4 241 735 B1**
(45) Date of publication and mention of the grant of the patent: **02.12.2026**
(21) Application number: 23171822.2
(22) Date of filing: 23.10.2017
(51) Int. Cl.: A61F 2/24

(54) **HEART VALVE PROSTHESIS FIXED THROUGH INTERVENTRICULAR SEPTUM**
DURCH INTERVENTRIKULÄRES SEPTUM BEFESTIGTE HERZKLAPPENPROTHESE
PROTHÈSE DE VALVULE CARDIAQUE FIXÉE À TRAVERS UN SEPTUM INTERVENTRICULAIRE

(30) Priority: 24.10.2016 CN 201610921114
(43) Date of publication of application: 13.09.2023
(62) Document number(s) of the earlier application(s) in accordance with Art. 76 EPC: 17863597.5 / 3 520 735
(73) Proprietor: Jenscare Scientific Co., Ltd., Ningbo, Zhejiang 315336 (CN)
(72) Inventor: LV, Shiwen, Ningbo, 315336 (CN); LI, Yibin, Ningbo, 315336 (CN); XU, Zhiyun, Ningbo, 315336 (CN); CHEN, Zhi, Ningbo, 315336 (CN); LU, Fanglin, Ningbo, 315336 (CN); LI, Jianan, Ningbo, 315336 (CN)
(74) Representative: ABG Intellectual Property Law, S.L.

(56) References cited:
- US-A1- 2012 101 571
- US-A1- 2015 127 097
- US-A1- 2016 038 280
- US-A1- 2016 120 643

## Description

The present application is a divisional application of Chinese patent application entitled "Heart Valve Prosthesis Anchored to Interventricular Septum and Conveying and Releasing Method Thereof", which is filed on October 24th, 2016 with the application number 201610921114.9.

### TECHNICAL FIELD

The present application relates to the field of medical equipment, and particularly relates to a heart valve prosthesis anchored to an interventricular septum and a conveying and releasing method thereof.

### BACKGROUND

Tricuspid valve is located at the right atrioventricular orifice. A common disease of the tricuspid valve is tricuspid insufficiency, that is, in the contraction period, the blood flows back from the right ventricle into the right atrium, which causes the excessive dilatation of the right atrium, increased pressure, and backflow disorder of venous blood. Because of the increasing load, a compensation on the right ventricle occurs. As a result, the right ventricle becomes fat and thick, and right heart failure easily happens.

Tricuspid regurgitation is generally caused by pulmonary arterial hypertension, right ventricular dilatation and tricuspid valve annulus dilatation. Clinically the expression of the etiologies (such as left heart failure and pulmonary arterial hypertension) of tricuspid regurgitation is common, and once tricuspid regurgitation arises, the symptoms of right heart failure such as fatigue, ascites, edema, hepatalgia, dyspepsia and anorexia are aggravated. Mild tricuspid regurgitation does not have obvious clinical symptoms, but operative treatment is required for serious tricuspid regurgitation.

The current clinical results indicate that, there are no ideal products for valve intervention and replacement of atrioventricular valve. The major reason is that, both of the mitral valve and the tricuspid valve have particular physiological structures, and the physiological environments under the valve annulus are complicated, which makes it difficult to accurately position and fix the products. The problems of the prior art are summarized as follows: (1) the conventional anchoring techniques mostly rely on the supporting force exerted on the atrioventricular valve annulus by the supporting frame; and doctors usually select a valve specification larger than the valve annulus of the autogenous atrioventricular valve of the patient, so as to conform to the contour of the mitral valve tissue, thus the huge supporting frame itself not only affects the outflow tract, but also easily presses the surrounding tissues, and further blocks the blood flow in the left ventricular outflow tract; (2) in the prior art, the supporting frame portion disposed in the atrium mostly configured to be a lattice, the huge supporting force of which easily presses the heart tissue; and (3) as for the mitral valve replacement, the supporting frame specification is too large, and the anterior valve of the mitral valve is easily pushed toward the left ventricular outflow tract; and the design of clamping the valve leaflet, which is introduced to fix the anterior valve of the mitral valve, makes the releasing step extremely complicated and be influenced by the calcification degree of the valve leaflet, which affects the success rate of the operation.

In conclusion, although the above described techniques individually have certain effects on the atrioventricular valve replacement, they still have defects. In the field of surgical treatment for valve lesion, a novel heart valve prosthesis is urgently needed to solve the above problems.

The U.S. patent publication No. US2012101571A1 discloses a heart valve prosthesis, comprising a housing component and a valve component. The housing component comprises a housing body having a housing passage extending there through. The housing body is configured to be located in, or adjacent to and communicating with, a native valve orifice of a heart and to engage structure of the heart to fix the housing body in relation to the valve orifice. The housing component is collapsible for delivery via catheter. The valve component comprises a valve body having a valve passage extending there through. The valve body is configured to be fixed within the housing passage with the valve passage extending along the housing passage. One or more flexible valve elements is/are secured to the valve body and extend across the valve passage for blocking blood flow in a first direction through the valve passage whilst allowing blood flow in the opposing direction. The valve component is collapsible for delivery via catheter separate to the housing component. An associated method of replacing a failed or failing heart valve utilising the heart valve prosthesis is also disclosed.

The U.S. patent publication No. US2015127097A1 discloses an implantable device for improving or rectifying a heart valve insufficiency, comprising a closure element which can be positioned in the passage area of a heart valve. in particular in the area between an atrium and a ventricle of the heart. and which has an upper, upstream end and a lower, downstream end, wherein at least one contact strip is arranged at least one of the ends of the closure element, which extends away from the closure element and is led back to the closure element in at least one loop, wherein at least a part of the extent of the strip can be placed against the inner wall of the heart.

### SUMMARY OF THE DISCLOSURE

The invention is set out in appended claims. The objective of the present application is to overcome the defects in the prior art. For the patients that require intervention valve replacement due to mitral valve or tricuspid valve insufficiency or stenosis, the present application provides a heart valve prosthesis anchored to an interventricular septum, and a conveying and releasing method of the heart valve prosthesis. The present application solves the problems of the anchoring technology in the prior art, which are caused by radially dilating the native valve annulus of the patient, and on the basis of ensuring the anchoring effects of the implanted valve, may reduce the influences on the outflow tract after the supporting frame is released, and avoids the traction for the valve annulus of the native valve.

An objective of the present application is achieved by the following technical solution:
a heart valve prosthesis anchored to an interventricular septum comprises a valve supporting frame and a fixing device; the valve supporting frame comprises a valve stitching section and an artificial valve; the artificial valve is fixedly connected to the valve stitching section; the fixing device comprises a fixing and supporting section and a fixing member; one end of the fixing and supporting section is connected to a proximal portion of the valve stitching section; and another end of the fixing and supporting section is connected to an interventricular septum of a patient by the fixing member to support the heart valve prosthesis and limit axial movement of the heart valve prosthesis.

The objectives of the present application may also be further realized by following technical solutions:
Preferably, the fixing and supporting section is provided with a curved section, which is configured to enable a proximal portion of the fixing and supporting section to cling to the interventricular septum of the patient. More preferably, the part of the fixing and supporting section that contacts the interventricular septum of the patient is a straight line section.

Preferably, the fixing and supporting section comprises a plurality of rods or wires; one end of each of the plurality of rods or wires is connected to the valve stitching section; and other ends of the plurality of rods or wires are mutually connected and are connected to the interventricular septum of the patient by the fixing member.

More preferably, on the circumferential circular arc where the outer edge of the valve stitching section is located, the largest arc length that is formed by the connection points between the plurality of rods or wires and the valve stitching section is greater than or equal to a quarter of the perimeter of the valve stitching section.

Preferably, the fixing and supporting section is formed by extending a skeleton of a proximal end of the valve stitching section.

In the invention, the fixing and supporting section is covered with a film.

Preferably, the fixing and supporting section is a triangular structure, or the fixing and supporting section is an arcuate structure, or the fixing and supporting section is a net-like structure. More preferably, the fixing and supporting section is provided therein with an enhancing rod.

Preferably, the fixing and supporting section and the fixing member are an integral structure, and the fixing member is barbs, or the fixing member is a sharp structure.

Preferably, the fixing device comprises a fixing member pushing system, and the fixing member pushing system pushes the fixing member so that one end of the fixing and supporting section is fixed on the interventricular septum of the patient.

In the invention, the fixing member is an anchoring needle, and the tail portion of the anchoring needle is provided with a stopper.

More preferably, the fixing member pushing system comprises a guide rail and a mandril; the guide rail is provided on the fixing and supporting section; the ends of the guide rail have a necking; the anchoring needle and the mandril are provided within the guide rail; by operating the mandril, the needle point portion of the anchoring needle passes through the guide rail and is inserted into the interventricular septum of the patient; and the diameter of the stopper is greater than the caliber of the necking.

More preferably, the stopper is provided with a connector, and the connector is a wire-shaped member; one end of the connector is connected to the fixing and supporting section, and the other end of the connector is connected to the stopper. Such a design mainly can ensure that the implanting instrument is detachably connected with the pushing system, thereby improving the accuracy of the needle inserting, and preventing the anchoring needle from deviating from the predetermined needle inserting point.

Preferably, the fixing and supporting section is an inverted cone-shaped structure; one end of the fixing and supporting section, which has a larger diameter, is connected to the proximal end of the valve stitching section; one end of the fixing and supporting section, which has a smaller diameter, is connected to a connecting rod; the connecting rod is rigid; a fixing member is arranged at the proximal portion of the connecting rod; and in a free state, the fixing member is fixed on the interventricular septum.

More preferably, the proximal portion of the connecting rod is a hollow tube; the tube wall is provided with an opening; and in a free state, the furthest distal end of the fixing member protrudes out of the opening on the hollow tube and inserts into the interventricular septum. The furthest distal end of the fixing member is sharp, and the distal portion of the fixing member is pre-shaped. The distal portion of the fixing member is pre-shaped to be one of or a combination of the following shapes: spiral, circle, arc, a combination of arc and straight line, branched double hooks, three-dimensional bent shape and multisection bent shape, and the distal end of the fixing member does not have a barb or has one or more barbs.

Preferably, the fixing member is a supporting frame having two larger end portions and a smaller middle portion, and is formed by a shape memory alloy.

Preferably, the heart valve prosthesis further comprises an auxiliary fixing device; one end of the auxiliary fixing device is connected to a distal end of the valve stitching section, and the other end of the auxiliary fixing device is fixed on an atrium tissue or fixed in a blood vessel of the patient.

More preferably, the auxiliary fixing device is a rod or a wire, or the auxiliary fixing device is a supporting frame.

Preferably, the heart valve prosthesis further comprises an auxiliary stabilizing device; a proximal end of the auxiliary stabilizing device is connected to the fixing and supporting section, or a proximal end of the auxiliary stabilizing device is connected to the fixing member, and a distal end of the auxiliary stabilizing device is connected to the valve stitching section.

More preferably, the auxiliary stabilizing device is a wire or a rod.

Preferably, in a longitudinal section parallel to a central axis of the artificial valve, the projections of the valve stitching section, of the fixing and supporting section and of the auxiliary stabilizing device are connected to become a closed structure.

In the invention, in a cross section perpendicular to a central axis of the artificial valve, a cross sectional area of the valve stitching section is less than a cross sectional area of a native valve annulus of the patient, which prevents the valve stitching section radially dilating the native valve annulus of the patient.

Preferably, the heart valve prosthesis further comprises a positioning ring; the positioning ring is connected to the valve stitching section, and in a free state, the positioning ring is disposed in an atrium of the patient.

Preferably, the positioning ring clings to a native valve annulus of the patient.

Preferably, and in a free state, a cross sectional area of the positioning ring is greater than a cross sectional area of a native valve annulus of the patient, and the positioning ring is capable of conforming to a non-uniform contour of an atrial wall or of the native valve annulus of the patient and not restricting a contraction function of the atrium.

Preferably, in a cross section perpendicular to a central axis of the artificial valve, the projection of the positioning ring is a ring-shaped structure; the ring-shaped structure comprises a circular structure, an elliptic structure or a D-shaped structure; and the valve stitching section is disposed in the positioning ring.

Preferably, in a cross section perpendicular to a central axis of the artificial valve, a center of the valve stitching section and a center of the positioning ring are not coincident.

More preferably, when the heart valve prosthesis is used for tricuspid valve intervention and replacement, the central axis of the valve stitching section deviates toward the cuspis medialis region of the tricuspid valve of the patient.

Preferably, in a longitudinal section parallel to a central axis of the artificial valve, the projection of the positioning ring is a disk-like structure or a bowl-like structure.

Preferably, the valve stitching section is a tube-like lattice structure, or the valve stitching section is a tube-like wave-shaped structure.

Preferably, a distal skeleton of the valve stitching section is provided with an extended section. Such a design enables the supporting frame to be controllably released. More preferably, the extension section and the valve stitching section are detachably connected. Such a design ensures that the extension section can be withdrawn from the human body while the supporting frame is ensured to be controllably released, thereby greatly reducing the implant, reducing the contact and stimulation to the atrium, and eliminating the limitation to the valve-in-valve implantation in future.

Preferably, the proximal skeleton of the valve stitching section is partially extended. Such a design enables the positioning ring to expand firstly while the proximal end of the supporting frame remains compressed, which facilitates adjusting the position of the supporting frame, thereby preventing the proximal end of the supporting frame from stabbing the blood vessel wall due to expanding in the adjusting process.

Preferably, the positioning ring has a skeleton made of a shape memory alloy; the skeleton is partially or entirely covered with a film, and the film material comprises metal material, polytetrafluoroethylene, polyethylene, polypropylene, terylene or animal-derived material.

More preferably, the skeleton includes a plurality of supporting rods; or the skeleton is a wave-shaped structure, a saw-shaped structure or a lattice structure that is formed by winding a metal memory material wire. The width of the supporting rods or the diameter of the metal memory material wire (for example, a nickel titanium alloy wire) is in the range of 0. 1-0.6mm.

Preferably, the positioning ring is provided with a barb, and in a free state, the barb is inserted into an autologous tissue of the patient.

Preferably, the positioning ring and the valve stitching section are made separately and independently and then are connected to form an integral structure.

Preferably, the positioning ring and the valve stitching section are an integral structure, and the positioning ring is formed by part of the rods in the skeleton of the valve stitching section.

Preferably, an outer surface of the valve stitching section is further provided with a filling device.

Preferably, the filling device has a skeleton made of a shape memory alloy; the skeleton is partially or entirely covered with a film; and the film material comprises metal material, polytetrafluoroethylene, polyethylene, polypropylene, terylene or animal-derived material.

Preferably, in a cross section perpendicular to a central axis of the artificial valve, the projection of the filling device is a ring-shaped structure, and the ring-shaped structure comprises a circular ring structure or a D-shaped ring structure.

Preferably, the filling device and the positioning ring are an integral structure.

Another objective of the present application is realized by the following technical solutions:
a method for conveying and releasing the heart valve prosthesis anchored to an interventricular septum comprises the following steps:
a. introducing a conveying conduit loaded with the heart valve prosthesis to an atrioventricular valve annulus via a minimally invasive incision in the atrial wall;
b. operating the conveying conduit, to release the fixing device;
c. operating the conveying conduit, to release the valve stitching section;
d. operating the conveying conduit, to make the fixing member be inserted into the interventricular septum of the patient; and
e. withdrawing the conveying conduit from a human body.

Preferably, the method further comprises the following steps between the step c and the step d:
c1. operating the conveying conduit, to partially release the valve stitching section and make the valve stitching section not be completely detached from the conveying conduit;
c2. operating the conveying conduit, to release the positioning ring, and positioning through the positioning ring; and
c3. operating the conveying conduit, to completely release the valve stitching section.

The advantages of the present application over the prior art are:
**1. In** the design of most of the products in the prior art, a supporting frame supports the valve annulus; what different from the prior art are that, in the present application, the fixing and supporting section is fixed on the interventricular septum of the patient by the fixing member, and such an anchoring mode enables the supporting frame to obtain a anchoring force large enough, without radially dilating the native valve annulus of the patient.
3. As different from the concentric structure of most of the conventional products in the prior art, in the present application, in a cross section perpendicular to a central axis of the artificial valve, a center of the valve stitching section and a center of the positioning ring are not coincident. When the heart valve prosthesis is used for mitral valve intervention and replacement, the central axis of the valve stitching section deviates toward the posterior valve region of the mitral valve of the patient, which can further reduce the block to the left ventricular outflow tract. When the heart valve prosthesis is used for tricuspid valve intervention and replacement, the central axis of the valve stitching section deviates toward the cuspis medialis region of the tricuspid valve of the patient, which facilitates the fixing and supporting section clinging to the target anchoring region, thereby obtaining a more ideal anchoring effect, and more stable movement of the valve.
4. What different from the design of most of the products in the prior art , in which a supporting frame supports the valve annulus, are that, in the present application, in a cross section perpendicular to a central axis of the artificial valve, the projection area of the valve stitching section is less than the projection area of the native valve annulus of the patient, which prevents the valve stitching section radially dilating the native valve annulus of the patient, thereby not only reducing the influence on the outflow tract after the supporting frame is released, and avoiding the traction to the protogenetic valve annulus, but also ensuring that the opening area of the valve will not change greatly because of the huge difference between the annulus of patients, and optimizing the performance of the valve. Moreover, the manufacturers may reduce the product specifications, which alleviate the goods stocking pressure of the manufacturers.
5. **In** the product in the prior art, the supporting frames located in the atrium mostly employs the lattice form, and the huge supporting force of the supporting frame easily presses the heart tissue, and the leak resistance effect is unsatisfying. What different from the supporting frame in the prior art are that, the positioning ring of the present application is disposed in the atrium of the patient and clings to the native valve annulus of the patient, and the positioning ring may conform to the non-uniform contour of the atrial wall or of the native valve annulus of the patient, thereby improving the leak resistance effect.
6. In the present application, a distal skeleton of the valve stitching section is provided with an extended section, and the extended section and the valve stitching section are detachably connected. Such a design ensures that the extended section may be withdrawn from the human body while the supporting frame is ensured to be controllably released, thereby greatly reducing the implant, reducing the contact and stimulation to the atrium, facilitating the conveying system being removed from the human body, and eliminating the limitation to the valve-in-valve implantation in future.

### BRIEF DESCRIPTION OF DRAWINGS

Figs. 1a-1c are schematic diagrams illustrating an embodiment of the present application.
Figs. 2a-2f are schematic diagrams illustrating multiple embodiments of the present application.
Figs. 3a-3d are schematic diagrams illustrating multiple embodiments of the present application.
Figs. 4a-4e are schematic diagrams illustrating multiple embodiments of the present application.
Figs. 5a-5d are schematic diagrams illustrating multiple embodiments of the present application.
Figs. 6a-6f are schematic diagrams illustrating multiple embodiments of the present application, wherein Fig. 6b is a sectional view of Fig. 6a.

### DETAILED DESCRIPTION

In order to make the objectives, the technical solutions and the advantages of the present application more apparent and better understood, the present application will be described in more details by referring to the accompanying figures and the embodiments.

In the present application, the distal end refers to the end far away from the cardiac apex, and the proximal end refers to the end near to the cardiac apex.

### Embodiment 1:

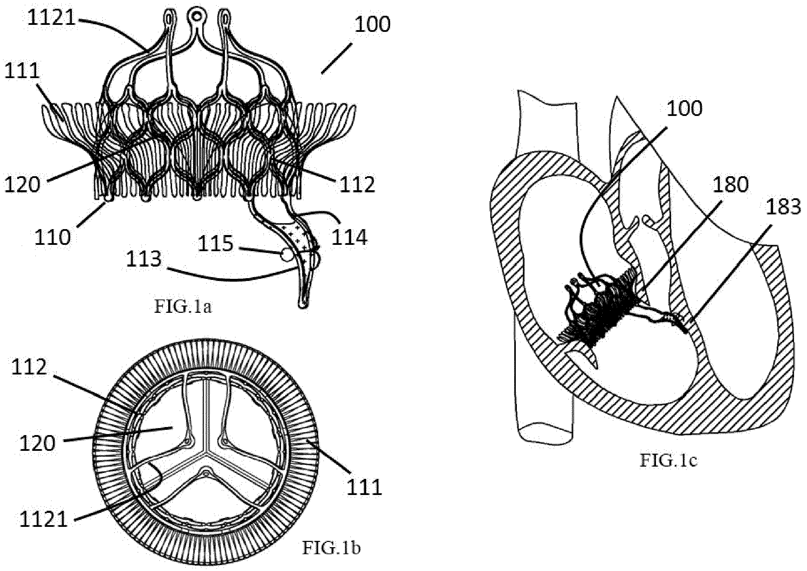

In an embodiment, as shown in Figs. 1a-1c, a heart valve prosthesis 100 anchored to an interventricular septum is provided for tricuspid valve intervention and replacement treatment, wherein the heart valve prosthesis comprises a valve supporting frame 110 and a fixing device 113; the valve supporting frame 110 comprises a valve stitching section 112 and an artificial valve 120; the valve stitching section 112 is a tube-like lattice structure; the artificial valve 120 is fixedly connected to the valve stitching section 112; the fixing device 113 comprises a fixing and supporting section 114 and a fixing member 115; one end of the fixing and supporting section 114 is connected to a proximal end portion of the valve stitching section 112, and the other end of the fixing and supporting section 114 is connected to the interventricular septum 183 of the patient by the fixing member 115, to support the heart valve prosthesis 100 and limit the axial movement of the heart valve prosthesis 100. The heart valve prosthesis 100 further comprises a positioning ring 111; the positioning ring 111 is connected to the valve stitching section 112, and in a free state, the positioning ring 111 is located in the atrium of the patient and clings to the native valve annulus 180 of the patient. In the design of most of the products in the prior art, the valve annulus is supported by a supporting frame; what different from the prior art are that, in the present application, the fixing and supporting section 114 is fixed to the interventricular septum 183 of the patient by the fixing member 115, and such an anchoring mode enables the valve supporting frame 110 to obtain a anchoring force large enough, without radially dilating the native valve annulus of the patient. The fixing and supporting section 114 is an extension of the proximal skeleton of the valve stitching section 112, and the fixing and supporting section 114 is rigid. In such a design, it is considered that the whole apparatus is supported in the target position by the fixing device 113, and the rigidity design may ensure the anchoring function. The distal skeleton of the valve stitching section 112 is provided with an extension section 1121, which enables the supporting frame to be controllably released, to improve the positioning accuracy.

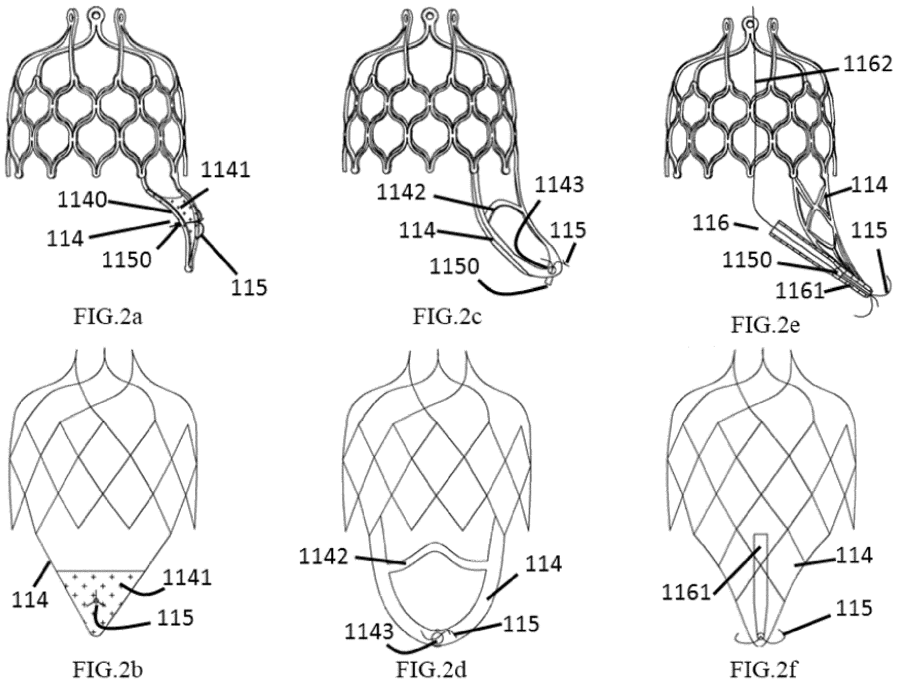

In an embodiment, as shown in Figs. 2a and 2b, the fixing and supporting section 114 is formed by a plurality of rods. One end of each of the plurality of rods is connected to the valve stitching section 112, and the other ends of the plurality of rods are mutually connected and are connected to the interventricular septum of the patient by the fixing member 115. The fixing and supporting section 114 is a triangular structure, the fixing and supporting section 114 is provided with a curved section 1140, thereby enabling the proximal end portion of the fixing and supporting section 114 to cling to the interventricular septum of the patient. The contact part of the fixing and supporting section 114 and the interventricular septum 183 of the patient is a straight line section. Such a design can increase the contact area between the fixing and supporting section 114 and the interventricular septum 183 of the patient to the utmost extent, thereby enhancing the anchoring effect. The fixing member 115 is an anchoring needle, and the distal portion of the anchoring needle 115 is pre-shaped. The needle point portion of the anchoring needle 115 is pre-shaped to be spiral, circular or arcuate, and the needle point portion of the anchoring needle 115 has a plurality of barbs. The tail portion of the anchoring needle 115 is provided with a stopper 1150, and the diameter of the stopper 1150 is greater than the needle diameter of the anchoring needle 115. The fixing and supporting section 114 is covered with a film 1141, and the material of the film 1141 comprises metal material, polytetrafluoroethylene, polyethylene, polypropylene, terylene or animal-derived material. The needle point portion of the anchoring needle 115 passes through the film 1141 and is inserted into the heart tissue of the patient.

In another embodiment, as shown in Figs. 2c and 2d, the fixing and supporting section 114 is an arcuate structure, and the fixing and supporting section 114 is provided therein with an enhancing rod 1142. The advantage of such a design is that the rigidity of the fixing and supporting section 114 is improved, thereby ensuring the anchoring function. The fixing member 115 is an anchoring needle, and the distal portion of the anchoring needle 115 is pre-shaped. The needle point portion of the anchoring needle 115 is pre-shaped to be a combination of arc line and straight line, or to be branched double hooks, and the needle point portion of the anchoring needle 115 has one barb. The tail portion of the anchoring needle 115 is provided with a stopper 1150. The fixing and supporting section 114 is provided with an opening 1143, and the needle point portion of the anchoring needle 115 passes through the opening 1143 and is inserted into the heart tissue of the patient. The diameter of the stopper 1150 is greater than the aperture of the opening 1143.

In another embodiment, as shown in Figs. 2e-2f, the fixing and supporting section 114 is a net-like structure. The fixing member 115 is an anchoring needle, the distal portion of the anchoring needle 115 is pre-shaped, and the needle point portion of the anchoring needle 115 is pre-shaped to be a three-dimensional bent shape or a multi-sectioned bent shape. The needle point portion of the anchoring needle 115 is not provided with any barbs, and the tail portion of the anchoring needle 115 is provided with a stopper 1150. The fixing device 113 comprises a fixing member pushing system 116, and the fixing member pushing system 116 pushes the fixing member 115, so that one end of the fixing and supporting section 114 is fixed on the interventricular septum of the patient. The fixing member pushing system 116 comprises a guide rail 1161 and a mandril 1162; the guide rail 1161 is fixedly provided on the fixing and supporting section 114; the ends of the guide rail 1161 have a necking; the anchoring needle 115 and the mandril 1162 are provided within the guide rail 1161; by operating the mandril 1162, the needle point portion of the anchoring needle 115 passes through the guide rail 1161 and is inserted into the interventricular septum of the patient; and the diameter of the stopper 1150 is greater than the aperture of the necking. After it is confirmed that the anchoring effect of the anchoring needle 115 is ideal, the mandril 1162 is withdrawn from the human body.

### Embodiment 2:

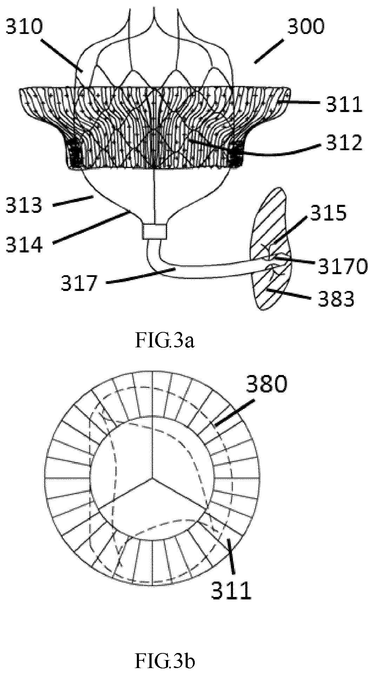

In an embodiment, as shown in Figs. 3a-3c, a heart valve prosthesis 300 anchored to an interventricular septum is provided for tricuspid valve intervention and replacement treatment, wherein the heart valve prosthesis comprises a valve supporting frame 310 and a fixing device 313; the valve supporting frame 310 comprises a valve stitching section 312 and an artificial valve (not shown); the artificial valve is fixedly connected to the valve stitching section 312; the valve stitching section 312 is a tube-like wave-shaped structure; the fixing device 313 comprises a fixing and supporting section 314 and a fixing member 315; the fixing and supporting section 314 is an inverted cone-shaped structure; the fixing and supporting section 314 includes a plurality of rods or wires; one end of the fixing and supporting section 314, which has a larger diameter, is connected to the proximal end of the valve stitching section 312 through well-known techniques such as stitching, clipping or welding; one end of the fixing and supporting section 314, which has a smaller diameter, is provided with a connecting rod 317; the connecting rod 317 is rigid; one end of the connecting rod 317 is connected to one end of the fixing and supporting section 314; the proximal portion of the connecting rod 317 is provided with the fixing member 315, and in a free state, the fixing member 315 is fixed on the interventricular septum 383 of the patient, to support the heart valve prosthesis 300 and limit the axial movement of the heart valve prosthesis 300. The heart valve prosthesis 300 further comprises a positioning ring 311, and the positioning ring 311 is connected to the valve stitching section 312.

The proximal portion of the connecting rod 317 is a hollow tube, and the tube wall is provided with an opening 3170. The furthest distal end of the fixing member 315 is sharp, and the distal portion of the fixing member 315 is pre-shaped. The distal end portion of the fixing member 315 is pre-shaped to be one of or a combination of the following shapes: spiral, circle, arc, a combination of arc and straight line, branched double hooks, three-dimensional bent shape, and multi-section bent shape. The distal end of the fixing member 315 does not have a barb, or has one or more barbs. In a free state, the furthest distal end of the fixing member 315 protrudes out of the opening 3170 in the hollow tube 317 and is inserted into the interventricular septum 383.

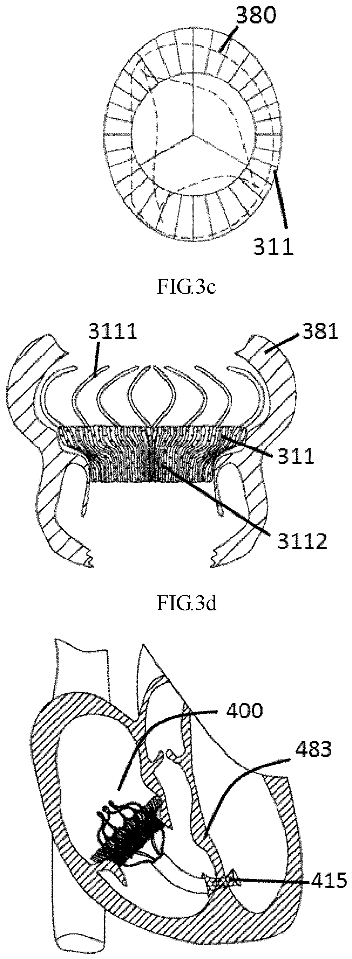

The positioning ring 311 is connected to the valve stitching section 312, and in a free state, the positioning ring 311 is disposed in the atrium of the patient and clings to the native valve annulus 380 of the patient. The cross sectional area of the positioning ring 311 is greater than the cross sectional area of the native valve annulus 380 (the dotted lines shown in Figs. 3b and 3c) of the patient, and the positioning ring 311 may conform to the non-uniform contour of the atrial wall or of the native valve annulus of the patient and does not restrict the contraction function of the atrium. In a cross section perpendicular to the central axis of the artificial valve, the projection of the positioning ring 311 is a ring-shaped structure, and the ring-shaped structure comprises a circular structure (Fig. 3b) or an elliptic structure (Fig. 3c).

In an embodiment, as shown in Fig. 3d, in a longitudinal section parallel to a central axis of the artificial valve, the projection of the positioning ring 311 is a bowl-like structure, and the bowl opening is supported within the atrium 381 of the patient. The positioning ring 311 has a skeleton 3111 made of a shape memory alloy, and the skeleton 3111 is partially covered with a film 3112, so as to prevent the positioning ring 311 supported within the atrial wall from blocking the coronary sinus, the superior vena cava and the inferior vena cava. The material of the film 3112 comprises metal material, polytetrafluoroethylene, polyethylene, polypropylene, terylene or animal-derived material.

### Embodiment 3:

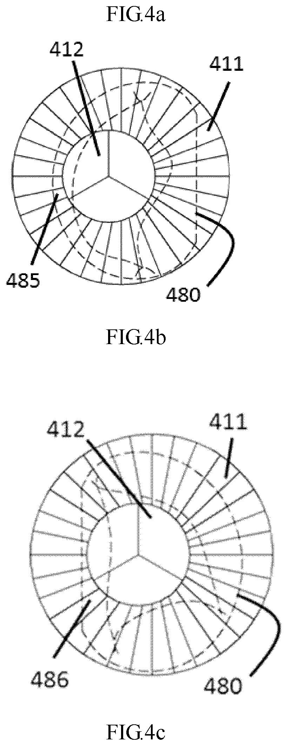

In an embodiment, as shown in Fig. 4a, a heart valve prosthesis 400 anchored to an interventricular septum is provided for tricuspid valve intervention and replacement treatment. This embodiment is different from embodiment 2 in that, the fixing member 415 is a supporting frame having two larger end portions and a smaller middle portion, and is formed by a shape memory alloy, and in a free state, the fixing member 415 is fixed to the interventricular septum 483.

In an embodiment, what different from the concentric structure of most of the conventional products are that: in the present application, in a cross section perpendicular to the central axis of the artificial valve, the projection of the positioning ring 411 is a circular ring-shaped structure; the valve stitching section 412 is disposed in the positioning ring 411; and the cross sectional area of the valve stitching section 412 is less than the cross sectional area of the native valve annulus 480 of the patient, which prevents the valve stitching section 412 radially dilating the native valve annulus 480 of the patient. Such a design not only reduces the influence on the outflow tract after the supporting frame is released, and avoids the traction to the protogenetic valve annulus, but also ensures that the opening area of the valve will not change greatly because of the huge difference between the valve annulus of patients, thereby optimizing the performance of the valve, and moreover, the manufacturers may reduce the product specifications, which alleviates the goods stocking pressure of the manufacturers. The center of the valve stitching section 412 and the center of the positioning ring 411 are not coincident, and the positioning ring 411 is eccentrically arranged relative to the valve stitching section 412. As shown in Fig. 4b, when the heart valve prosthesis 400 is used for mitral valve intervention and replacement, the central axis of the valve stitching section 412 deviates toward the posterior valve region 485 (shown by dotted lines) of the mitral valve of the patient, which can further reduce the block to the left ventricular outflow tract. As shown in Fig. 4c, when the heart valve prosthesis 400 is used for tricuspid valve intervention and replacement, the central axis of the valve stitching section 412 deviates toward the cuspis medialis region 486 (shown by dotted lines) of the tricuspid valve of the patient, which facilitates the fixing device clinging to the target anchoring region, thereby obtaining a more ideal anchoring effect, and more stable movement of the valve.

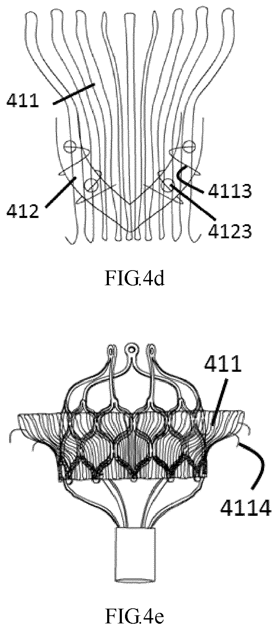

In an embodiment, as shown in Figs. 4d and 4e, the positioning ring 411 and the valve stitching section 412 are made separately and independently and then connected to form an integral structure. The skeleton of the valve stitching section 412 is provided with an opening 4123, and the positioning ring 411 passes through the opening 4123 and is connected to the valve stitching section 412 with a suture 4113. The positioning ring 411 has a skeleton made of a shape memory alloy, and the skeleton is entirely covered with a film. In a longitudinal section parallel to a central axis of the artificial valve, the projection of the positioning ring 411 is a disk-like structure. The positioning ring 411 is provided with a barb 4114, and when the positioning ring 411 clings to the native valve annulus of the patient, the barb 4114 is inserted into the autologous tissue of the patient.

### Embodiment 4:

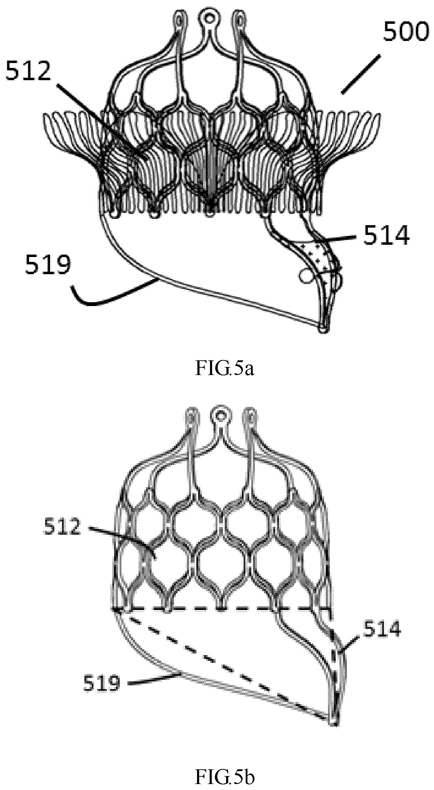

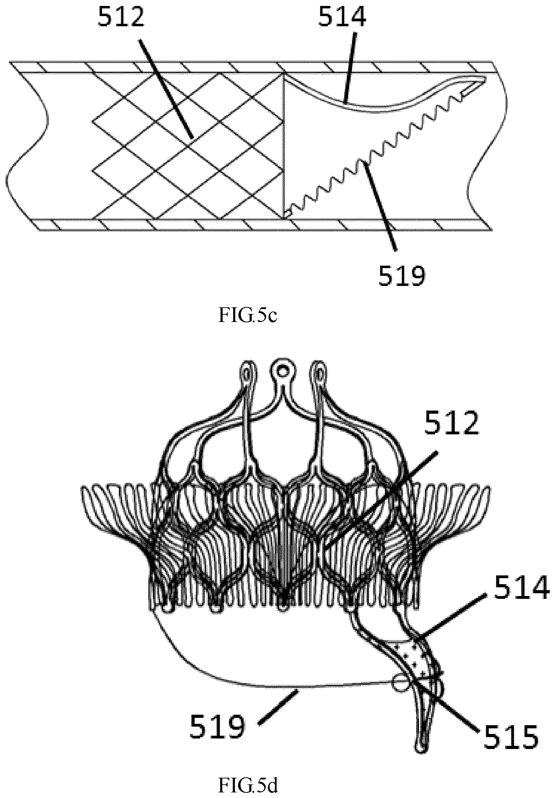

In an embodiment, as shown in Fig. 5a, the heart valve prosthesis 500 further comprises an auxiliary stabilizing device 519. The proximal end of the auxiliary stabilizing device 519 is connected to the fixing and supporting section 514, and the distal end of the auxiliary stabilizing device 519 is connected to the valve stitching section 512. In a longitudinal section parallel to a central axis of the artificial valve, the projections of the valve stitching section 512, of the fixing and supporting section 514 and of the auxiliary stabilizing device 519 are connected to become a closed structure, which enables the implant to be more secure in the target position while ensuring the operation convenience of fixing on one side, thereby preventing the implant from losing balance in the body of the patient. As shown in Fig. 5b, in order to maximize the above balancing effect, the distal end of the auxiliary stabilizing device 519 is connected to the position with the maximum diameter of the valve stitching section 512. In a longitudinal section parallel to a central axis of the artificial valve, the projections of the valve stitching section 512, of the fixing and supporting section 514 and of the auxiliary stabilizing device 519 are connected to become a triangle. In consideration of the uniformity of the length of the compressed fixing and supporting section 514 and the length of the compressed the auxiliary stabilizing device 519, as shown in Fig. 5c, the auxiliary stabilizing device 519 is designed to be a wire or a wave-like rod. In another embodiment, as shown in Fig. 5d, the proximal end of the auxiliary stabilizing device 519 is connected to the fixing member 515, and the distal end of the auxiliary stabilizing device 519 is connected to the valve stitching section 512.

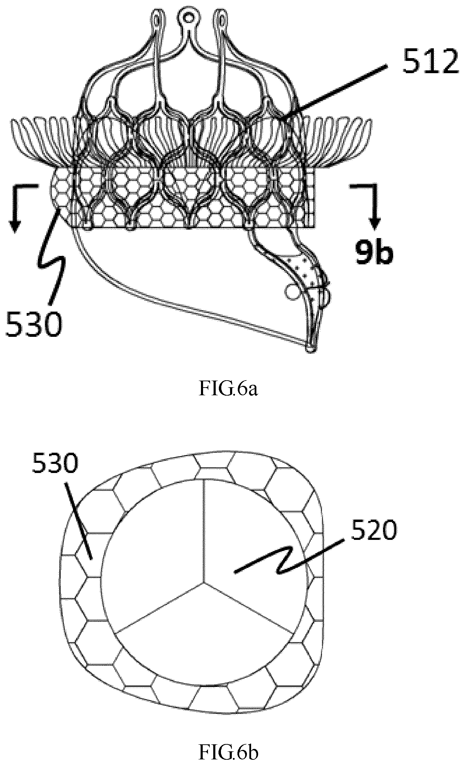

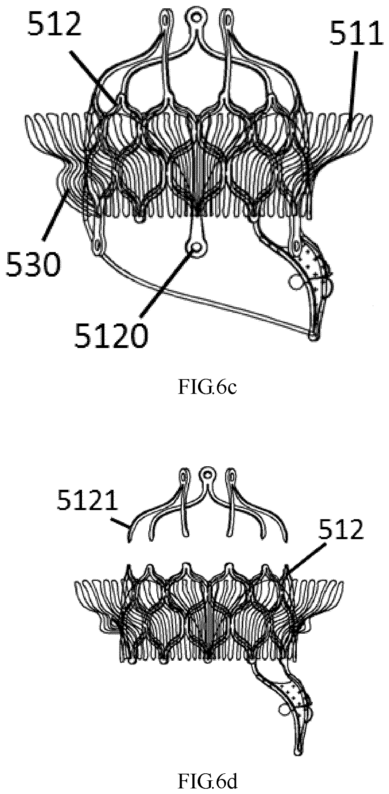

In another embodiment, as shown in Fig. 6a, an outer surface of the valve stitching section 512 is further provided with a filling device 530. In a cross section perpendicular to the central axis of the artificial valve 520, as shown in Fig. 6b, the projection of the filling device 530 is a ring-shaped structure, and the ring-shaped structure comprises a circular ring structure or a D-shaped ring structure. The advantage of such a design is that the contact with the native valve is increased, thereby improving the leak resistance effect. The filling device 530 has a skeleton made of a shape memory alloy, the skeleton is partially or entirely covered with a film, and the film material comprises metal material, polytetrafluoroethylene, polyethylene, polypropylene, terylene or animal-derived material. In another embodiment, as shown in Fig. 6c, the filling device 530 and the positioning ring 511 are an integral structure. The proximal skeleton 5120 of the valve stitching section 512 is partially extended. Such a design enables the positioning ring 511 to expand firstly while the proximal end of the valve stitching section 512 remains compressed, which facilitates adjusting the position of the supporting frame, thereby preventing the proximal end of the supporting frame from stabbing the blood vessel wall due to expanding in the adjusting process.

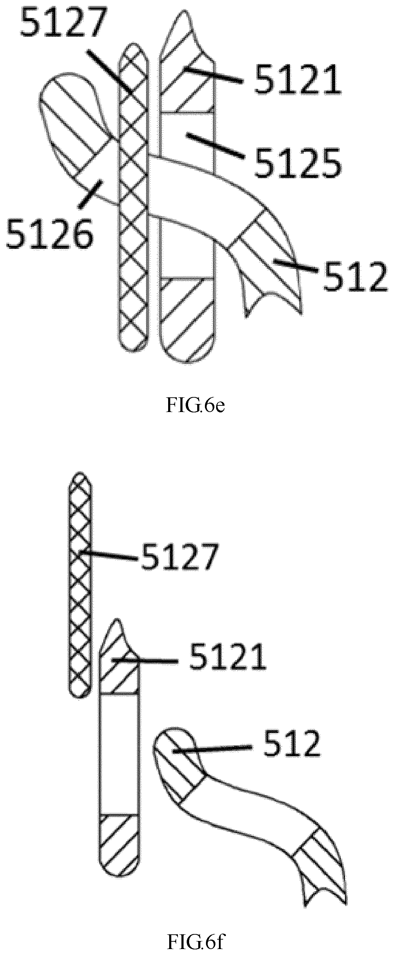

In another embodiment, as shown in Figs. 6d-6f, the distal skeleton of the valve stitching section 512 is provided with an extended section 5121, and the extended section 5121 and the valve stitching section 512 are detachably connected. Such a design ensures that the extended section 5121 may be withdrawn from the human body while the supporting frame is ensured to be controllably released, thereby greatly reducing the implant, reducing the contact and stimulation to the atrium, facilitating the conveying system being removed from the human body, and eliminating the limitation to the valve-in-valve implantation in future. As shown in Fig. 6e, the proximal end of the extended section 5121 is provided with a hole-like structure 5125; the distal skeleton of the valve stitching section 512 enters the hole-like structure 5125 in a staggered way; the distal skeleton of the valve stitching section 512 is provided with a locking hole 5126, and the locking is realized by inserting a locking rod 5127 into the locking hole 5126. As shown in Fig. 6f, when the locking rod 5127 is pulled out of the locking hole 5126, the distal skeleton of the valve stitching section 512 is separated from the hole-like structure 5125 of the extended section 5121, to realize the detaching of the extension section 5121 and the valve stitching section 512.

## Claims

1. A heart valve prosthesis configured to be anchored to an interventricular septum, wherein
the heart valve prosthesis (100, 500) comprises a valve supporting frame (110) and a fixing device (113); the valve supporting frame (110) comprises a valve stitching section (112) and an artificial valve (120, 520); the artificial valve (120, 520) is fixedly connected to the valve stitching section (112, 512); the fixing device (113) comprises a fixing and supporting section (114, 514) and a fixing member (115, 515); one end of the fixing and supporting section (114, 514) is connected to a proximal portion of the valve stitching section (112, 512); and another end of the fixing and supporting section (114, 514) is configured to be connected to the interventricular septum (183) of a patient by the fixing member (115, 515), to support the heart valve prosthesis (100, 500) and limit axial movement of the heart valve prosthesis (100, 500);
wherein in a cross-section perpendicular to a central axis of the artificial valve (120, 520), a cross-sectional area of the valve stitching section (112, 512) is less than a cross-sectional area of a native valve annulus of the patient, which prevents the valve stitching section (112, 512) from radially dilating the native valve annulus of the patient;
the heart valve prosthesis being **characterized in that** the fixing and supporting section (114, 514) is covered with a film (1141); and
the fixing member (115, 515) is an anchoring needle, and a tail portion of the anchoring needle is provided with a stopper (1150).

2. The heart valve prosthesis configured to be anchored to the interventricular septum according to claim 1, wherein the fixing and supporting section (114, 514) is provided with a curved section, which is configured to enable a proximal portion of the fixing and supporting section (114, 514) to cling to the interventricular septum (183) of the patient.

3. The heart valve prosthesis configured to be anchored to the interventricular septum according to claim 1, wherein the fixing and supporting section (114, 514) comprises a plurality of rods or wires; one end of each of the plurality of rods or wires is connected to the valve stitching section (112, 512); and other ends of the plurality of rods or wires are mutually connected and configured to be connected to the interventricular septum (183) of the patient by the fixing member (115, 515).

4. The heart valve prosthesis configured to be anchored to the interventricular septum according to claim 1, wherein the fixing and supporting section (114, 514) is formed by extending a skeleton of a proximal end of the valve stitching section (112, 512).

5. The heart valve prosthesis configured to be anchored to the interventricular septum according to claim 1, wherein the fixing device (113) comprises a fixing member pushing system (116); and the fixing member pushing system (116) is configured to push the fixing member (115) so that one end of the fixing and supporting section (114, 514) is fixed on the interventricular septum (183) of the patient.

6. The heart valve prosthesis configured to be anchored to the interventricular septum according to claim 1, wherein a filling device (530) is arranged on an outer surface of the valve stitching section;
in a cross-section perpendicular to the central axis of the artificial valve (520), a projection of the filling device (530) is a ring-shaped structure, and the ring-shaped structure comprises a circular ring structure or a D-shaped ring structure; such that the contact with the native valve is increased, thereby improving the leak resistance effect.

7. The heart valve prosthesis configured to be anchored to the interventricular septum according to any one of claims 1-6, wherein the heart valve prosthesis (100, 500) further comprises a positioning ring (111, 511); the positioning ring (111, 511) is connected to the valve stitching section (112, 512); and in a free state, the positioning ring (111, 511) is disposed in an atrium of the patient; and the positioning ring (111, 511) clings to the native valve annulus of the patient.

8. The heart valve prosthesis configured to be anchored to the interventricular septum according to claim 7, wherein in a free state, a cross-sectional area of the positioning ring (111, 511) is greater than a cross-sectional area of the native valve annulus of the patient; and the positioning ring (111, 511) is capable of conforming to a non-uniform contour of an atrial wall or of the native valve annulus of the patient and not restricting a contraction function of the atrium.

9. The heart valve prosthesis configured to be anchored to the interventricular septum according to claim 8, wherein in a cross-section perpendicular to a central axis of the artificial valve, a center of the valve stitching section and a center of the positioning ring are not coincident.

## Patentansprüche

1. Herzklappenprothese, die zur Verankerung am Ventrikelseptum ausgebildet ist,
wobei die Herzklappenprothese (100, 500) einen Klappenstützrahmen (110) und eine Befestigungsvorrichtung (113) umfasst; der Klappenstützrahmen (110) einen Klappennähabschnitt (112, 512) und eine künstliche Klappe (120, 520) umfasst; die künstliche Klappe (120, 520) fest mit dem Klappennähabschnitt (112, 512) verbunden ist; die Befestigungsvorrichtung (113) einen Befestigungs- und Stützabschnitt (114, 514) und ein Befestigungselement (115, 515) umfasst; ein Ende des Befestigungs- und Stützabschnitts (114, 514) mit einem proximalen Abschnitt des Klappennähabschnitts (112, 512) verbunden ist; und das andere Ende des Befestigungs- und Stützabschnitts (114, 514) dazu ausgebildet ist, mittels des Befestigungselements (115, 515) am Ventrikelseptum (183) eines Patienten verankert zu werden, um die Herzklappenprothese (100, 500) zu stützen und eine axiale Bewegung der Herzklappenprothese (100, 500) zu begrenzen;
wobei in einem senkrecht zu einer Zentralachse der künstlichen Klappe (120, 520) verlaufenden Querschnitt eine Querschnittsfläche des Klappennähabschnitts (112, 512) kleiner ist als eine Querschnittsfläche eines nativen Klappenrings des Patienten, wodurch verhindert wird, dass der Klappennähabschnitt (112, 512) den nativen Klappenring des Patienten radial aufweitet;
**dadurch gekennzeichnet, dass**
der Befestigungs- und Stützabschnitt (114, 514) mit einer Folie (1141) bedeckt ist; und
das Befestigungselement (115, 515) eine Verankerungsnadel ist und ein Endabschnitt der Verankerungsnadel mit einem Anschlag (1150) versehen ist.

2. Herzklappenprothese, die zur Verankerung am Ventrikelseptum ausgebildet ist, nach Anspruch 1, **dadurch gekennzeichnet, dass** der Befestigungs- und Stützabschnitt (114, 514) mit einem gekrümmten Abschnitt versehen ist, der dazu ausgebildet ist, einen proximalen Abschnitt des Befestigungs- und Stützabschnitts (114, 514) am Ventrikelseptum (183) des Patienten zur Anlage zu bringen.

3. Herzklappenprothese, die zur Verankerung am Ventrikelseptum ausgebildet ist, nach Anspruch 1, **dadurch gekennzeichnet, dass** der Befestigungs- und Stützabschnitt (114, 514) eine Vielzahl von Stäben oder Drähten umfasst; ein erstes Ende jedes der Vielzahl von Stäben oder Drähten mit dem Klappennähabschnitt (112, 512) verbunden ist; und ein zweites Ende jedes der Vielzahl von Stäben oder Drähten dazu ausgebildet ist, miteinander verbunden zu sein und mittels des Befestigungselements (115, 515) mit dem Ventrikelseptum (183) des Patienten verbunden zu werden.

4. Herzklappenprothese, die zur Verankerung am Ventrikelseptum ausgebildet ist, nach Anspruch 1, **dadurch gekennzeichnet, dass** der Befestigungs- und Stützabschnitt (114, 514) durch Fortsetzung des Skeletts eines proximalen Endes des Klappennähabschnitts (112, 512) gebildet ist.

5. Herzklappenprothese, die zur Verankerung am Ventrikelseptum ausgebildet ist, nach Anspruch 1, **dadurch gekennzeichnet, dass** die Befestigungsvorrichtung (113) ein Befestigungselement-Vorschubsystem (116) umfasst; und das Befestigungselement-Vorschubsystem (116) dazu ausgebildet ist, das Befestigungselement (115) vorzuschieben, sodass ein Ende des Befestigungs- und Stützabschnitts (114, 514) am Ventrikelseptum (183) des Patienten befestigt wird.

6. Herzklappenprothese, die zur Verankerung am Ventrikelseptum ausgebildet ist, nach Anspruch 1, **dadurch gekennzeichnet, dass** eine Füllvorrichtung (530) an einer Außenoberfläche des Klappennähabschnitts angeordnet ist;
wobei in einem senkrecht zu der Zentralachse der künstlichen Klappe (520) verlaufenden Querschnitt eine Projektion der Füllvorrichtung (530) eine ringförmige Struktur ist und die ringförmige Struktur eine kreisringförmige Struktur oder eine D-förmige Ringstruktur umfasst; sodass der Kontakt mit der nativen Klappe vergrößert wird, wodurch die Dichtwirkung gegen Leckagen verbessert wird.

7. Herzklappenprothese, die zur Verankerung am Ventrikelseptum ausgebildet ist, nach einem der Ansprüche 1-6, **dadurch gekennzeichnet, dass** die Herzklappenprothese (100, 500) ferner einen Positionierungsring (111, 511) umfasst; der Positionierungsring (111, 511) mit dem Klappennähabschnitt (112, 512) verbunden ist; und der Positionierungsring (111, 511) in einem Freizustand in einem Atrium des Patienten angeordnet ist; und der Positionierungsring (111, 511) an dem nativen Klappenring des Patienten anliegt.

8. Herzklappenprothese, die zur Verankerung am Ventrikelseptum ausgebildet ist, nach Anspruch 7, **dadurch gekennzeichnet, dass** in einem Freizustand eine Querschnittsfläche des Positionierungsrings (111, 511) größer ist als eine Querschnittsfläche des nativen Klappenrings des Patienten; und der Positionierungsring (111, 511) in der Lage ist, sich einer ungleichmäßigen Kontur einer Atriumwand oder des nativen Klappenrings des Patienten anzupassen, ohne eine Kontraktionsfunktion des Atriums einzuschränken.

9. Herzklappenprothese, die zur Verankerung am Ventrikelseptum ausgebildet ist, nach Anspruch 8, **dadurch gekennzeichnet, dass** in einem senkrecht zu einer Zentralachse der künstlichen Klappe verlaufenden Querschnitt ein Mittelpunkt des Klappennähabschnitts und ein Mittelpunkt des Positionierungsrings nicht zusammenfallen.

## Revendications

1. Prothèse de valve cardiaque configurée pour être ancrée à un septum interventriculaire, dans laquelle
la prothèse de valve cardiaque (100, 500) comprend une structure de support de valve (110) et un dispositif de fixation (113); la structure de support de valve (110) comprend une section de suture de valve (112) et une valve artificielle (120, 520); la valve artificielle (120, 520) est reliée de manière fixe à la section de suture de valve (112, 512); le dispositif de fixation (113) comprend une section de fixation et de support (114, 514) et un élément de fixation (115, 515); une extrémité de la section de fixation et de support (114, 514) est reliée à une partie proximale de la section de suture de valve (112, 512); et une autre extrémité de la section de fixation et de support (114, 514) est configurée pour être reliée au septum interventriculaire (183) d'un patient par l'élément de fixation (115, 515), pour supporter la prothèse de valve cardiaque (100, 500) et limiter le mouvement axial de la prothèse de valve cardiaque (100, 500);
dans laquelle dans une section transversale perpendiculaire à un axe central de la valve artificielle (120, 520), une surface de section transversale de la section de suture de valve (112, 512) est inférieure à une surface de section transversale d'un anneau valvulaire natif du patient, ce qui empêche la section de suture de valve (112, 512) de dilater radialement l'anneau valvulaire natif du patient;
la prothèse de valve cardiaque étant **caractérisée en ce que**
la section de fixation et de support (114, 514) est recouverte d'un film (1141); et
l'élément de fixation (115, 515) est une aiguille d'ancrage, et une partie de queue de l'aiguille d'ancrage est munie d'une butée (1150).

2. Prothèse de valve cardiaque configurée pour être ancrée à un septum interventriculaire selon la revendication 1, dans laquelle la section de fixation et de support (114, 514) est munie d'une section courbe, qui est configurée pour permettre à une partie proximale de la section de fixation et de support (114, 514) d'épouser le septum interventriculaire (183) du patient.

3. Prothèse de valve cardiaque configurée pour être ancrée à un septum interventriculaire selon la revendication 1, dans laquelle la section de fixation et de support (114, 514) comprend une pluralité de tiges ou de fils; une extrémité de chacune de la pluralité de tiges ou de fils est reliée à la section de suture de valve (112, 512); et les autres extrémités de la pluralité de tiges ou de fils sont mutuellement reliées et configurées pour être reliées au septum interventriculaire (183) du patient par l'élément de fixation (115, 515).

4. Prothèse de valve cardiaque configurée pour être ancrée à un septum interventriculaire selon la revendication 1, dans laquelle la section de fixation et de support (114, 514) est formée en prolongeant un squelette d'une extrémité proximale de la section de suture de valve (112, 512).

5. Prothèse de valve cardiaque configurée pour être ancrée à un septum interventriculaire selon la revendication 1, dans laquelle le dispositif de fixation (113) comprend un système de poussée d'élément de fixation (116); et le système de poussée d'élément de fixation (116) est configuré pour pousser l'élément de fixation (115) de sorte qu'une extrémité de la section de fixation et de support (114, 514) soit fixée sur le septum interventriculaire (183) du patient.

6. Prothèse de valve cardiaque configurée pour être ancrée à un septum interventriculaire selon la revendication 1, dans laquelle un dispositif de remplissage (530) est agencé sur une surface externe de la section de suture de valve;
dans une section transversale perpendiculaire à l'axe central de la valve artificielle (520), une projection du dispositif de remplissage (530) est une structure en forme d'anneau, et la structure en forme d'anneau comprend une structure d'anneau circulaire ou une structure d'anneau en forme de D; de telle sorte que le contact avec la valve native soit augmenté, améliorant ainsi l'effet de résistance aux fuites.

7. Prothèse de valve cardiaque configurée pour être ancrée au septum interventriculaire selon l'une quelconque des revendications 1 à 6, dans laquelle la prothèse de valve cardiaque (100, 500) comprend en outre un anneau de positionnement (111, 511); l'anneau de positionnement (111, 511) est relié à la section de suture de valve (112, 512); et à l'état libre, l'anneau de positionnement (111, 511) est disposé dans une oreillette du patient; et l'anneau de positionnement (111, 511) épouse l'anneau valvulaire natif du patient.

8. Prothèse de valve cardiaque configurée pour être ancrée à un septum interventriculaire selon la revendication 7, dans laquelle à l'état libre, une surface de section transversale de l'anneau de positionnement (111, 511) est supérieure à une surface de section transversale de l'anneau valvulaire natif du patient; et l'anneau de positionnement (111, 511) est capable de se conformer à un contour non uniforme d'une paroi auriculaire ou de l'anneau valvulaire natif du patient et de ne pas restreindre une fonction de contraction de l'oreillette.

9. Prothèse de valve cardiaque configurée pour être ancrée à un septum interventriculaire selon la revendication 8, dans laquelle dans une section transversale perpendiculaire à un axe central de la valve artificielle, un centre de la section de suture de valve et un centre de l'anneau de positionnement ne coïncident pas.
